# EUROPÄISCHE PATENTSCHRIFT

(11) **EP 1 054 913 B1**
(45) Veröffentlichungstag und Bekanntmachung des Hinweises auf die Patenterteilung: **23.04.2003**
(21) Anmeldenummer: 99910218.9
(22) Anmeldetag: 11.02.1999
(51) Int. Cl.: C08F 26/00, C08F 293/00, A61K 35/16

(54) **VERFAHREN ZUR HERSTELLUNG VON POLYMEREN AUS N-VINYLVERBINDUNGEN**
METHOD FOR THE PRODUCTION OF POLYMERS FROM N-VINYL COMPOUNDS
PROCEDE DE PRODUCTION DE POLYMERES A PARTIR DE COMPOSES N-VINYLIQUES

(30) Priorität: 19.02.1998 DE 19806853
(43) Veröffentlichungstag der Anmeldung: 29.11.2000
(73) Patentinhaber: BASF AKTIENGESELLSCHAFT, 67056 Ludwigshafen (DE); MAX-PLANCK-GESELLSCHAFT ZUR FÖRDERUNG DER WISSENSCHAFTEN E.V., 14195 Berlin (DE)
(72) Erfinder: RAETHER, Roman, Benedikt, D-67117 Limburgerhof (DE); PAULUS, Wolfgang, D-55128 Mainz (DE); BRAUN, Frank, D-67150 Niederkirchen (DE); MÜLLEN, Klaus, D-50939 Köln (DE); KLAPPER, Markus, D-55128 Mainz (DE); STEENBOCK, Marco, D-55126 Mainz (DE)
(74) Vertreter: Kinzebach, Werner, Dr.
(86) Internationale Anmeldenummer: EP9900891
(87) Internationale Veröffentlichungsnummer: WO99042501

(56) Entgegenhaltungen:
- EP-A- 0 135 280
- WO-A-94/11412
- WO-A-94/13706

## Beschreibung

Die vorliegende Erfindung betrifft ein Verfahren zur Herstellung von Polymeren aus N-Vinylverbindungen. Des weiteren betrifft die vorliegende Erfindung die Verwendung von Radikalen zur Herstellung von Polymeren aus N-Vinylverbindungen. Darüberhinaus betrifft die vorliegende Erfindung die nach dem erfindungsgemäßen Verfahren erhältlichen Polymeren, sowie deren Verwendung.

Ein gebräuchliches Verfahren zur Polymerisation von N-Vinylverbindungen wie 1-Vinyl-2-pyrrolidon (N-Vinylpyrrolidon, nachfolgend NVP) oder N-Vinylformamid (nachfolgend NVF) ist die radikalische Polymerisation. Sie erlaubt auch die Copolymerisation der N-Vinylverbindungen mit anderen Monomeren. Wegen unvermeidbarer Nebenreaktionen wie Kettenübertragung, Disproportionierung, Rekombination oder Eliminierung ist jedoch eine Kontrolle der Molekulargewichtsverteilung nur sehr schwer möglich. Normalerweise werden Polymere mit einem Polydispersitätsindex PDI von 2,0 oder größer erhalten. PDI ist dabei definiert als PDI = M_{w}/Mₙ, wobei M_{w} das Gewichtsmittel und Mₙ das Zahlenmittel des Molekulargewichts sind. Auch die Architektur und Struktur der Polymeren läßt sich nur schlecht beeinflussen.

Daher wurde zur Herstellung von Polymeren mit enger Molekulargewichtsverteilung die sogenannte "kontrollierte radikalische Polymerisation", die manchmal auch "lebende radikalische Polymerisation" genannt wird, entwickelt, die beispielsweise in M.K. Georges et al, Trends in Polymer Science, Vol. 2, No. 2 (1994), Seite 66 bis 72 beschrieben wird. Die grundlegende Strategie dieser Methode besteht darin, die reaktiven radikalischen Kettenenden einer wachsenden Polymerkette zeitweise zu blockieren und wieder gezielt zu reaktivieren (Reinitiierung). Das dynamische Gleichgewicht zwischen aktiver und schlafender Form führt zu einer geringen stationären Konzentration freier Polymerradikale.

In der EP-A 135 280 wird die Verwendung stabiler N-Oxylradikale beschrieben, die reversibel mit den reaktiven Kettenenden kombinieren. Nach diesem Verfahren werden jedoch keine hochmolekularen Polymere, sondern nur Oligomere erhalten.

Eine besondere Gruppe von Initiatoren zur kontrollierten radikalischen Polymerisation stellen Verbindungen dar, die in Radikal starter und N-Oxylradikale gespalten werden konnen (Trends in Polymer Science, 4(6), 1996, 183 - 188). Diese Verbindungen ermöglichen beispielsweise den Aufbau verzweigter Polymerer. Allerdings können nur ausgewählte Monomere polymerisiert werden und die Reaktionstemperaturen sind unbefriedigend hoch.

Im allgemeinen sind die Reaktionsgeschwindigkeiten bei der Polymerisation von Monomeren in Gegenwart von N-Oxylradikalen für viele technische Zwecke zu gering. Daher wurden z.B. starke organische Säuren mitverwendet (US-A 5,322,912). Diese können jedoch bei der Aufarbeitung der Produkte Schwierigkeiten verursachen.

In der DE-OS 195 16 967 werden Verfahren beschrieben, bei denen vinylische Monomeren in Gegenwart gängiger Radikalstarter und Elektronendonoren, die das radikalische Kettenende stabilisieren, polymerisiert werden.

In der WO 94/18241 ist die Polymerisation von NVP mit mehreren Initiatoren, die eine unterschiedliche Zersetzungstemperatur aufweisen, beschrieben. Eine solche Regelung ist umständlich, und die erhaltenen Polymere sind nicht mit funktionellen Gruppen terminiert, die für eine Reinitiierung genutzt werden können.

Matyjaszewski beschreibt in Polym. Mater. Sci. Eng., Vol. 76, S. 147-148 (1997) die kontrollierte radikalsiche Polymerisation von NVP durch Atom Transfer Radical Polymerization (ATRP). Für die ATRP-Methode werden allerdings Schwermetalle benötigt. Das Reduktionsprodukt Poly-NVP ist ein guter Komplexbildner für diese Schwermetalle, weshalb das durch ATRP hergestellte Poly-NVP schwermetallhaltig und daher für eine Verwendung in der Medizin ungeeignet ist.

Polymere, die unter Verwendung eines Reglers radikalisch polymerisiert wurden, lassen sich durch Reinitiierung in Gegenwart eines weiteren Monomeren zu Blockcopolymeren umsetzen. Abadie beschreibt in Eur. Polym. J., Vol. 26(5), S. 515-520 (1990) daß Blockcopolymere enthaltend NVP prinzipiell auch durch einen Wechsel des Reaktionsmechanismus erhältlich sind. Dieses Verfahren hat den Nachteil, daß ein zusätzlicher Reaktionsschritt erforderlich ist im Vergleich zu einem rein radikalischen Verfahren.

Turner offenbart in Polym. Reprints (Am. Chem. Soc., Div. Polym. Chem.) (1988), Vol. 29(2), S, 6-7 die Synthese eines Blockcopolymeren aus NVP und Styrol, bei der jedoch die Molmasse des Produktes geringer ist als die des Ausgangspolymeren.

Munam Lee beschreibt in J. Chem. Coc. Faraday Trans. 1, Vol. 74(7), S. 1738-1749 (1978) eine kontrollierte radikalische Polymerisation von NVP. Die Polymerisation verläuft jedoch sehr langsam: nach 40 Stunden Reaktionszeit werden nur "Spuren" von Polymer gefunden.

Als Regler für die radikalische Polymerisation können auch Triazolylradikale verwendet werden, wie die ältere, nicht vorveröffentlichte Anmeldung DE-P 19636996.7 lehrt. Die Anmeldung nennt als bevorzugte Monomere Vinylaromaten, Alkylester der Acrylsäure und Methacrylsäure, und Acrylnitril. Homopolymere und statistische Copolymere aus N-Vinylverbindungen werden nicht erwähnt.

Aufgabe der vorliegenden Erfindung war es, ein neues Verfahren zur Herstellung von Polymeren enthaltend N-Vinylverbindungen wie NVP und NVF zur Verfügung zu stellen, das die vorgenannten Nachteile nicht aufweist. Des weiteren sollte das neue Verfahren eine sehr gute Kontrolle sowohl über die Molekulargewichtsverteilung als auch die Architektur und Struktur der Polymeren erlauben. Darüberhinaus sollte ein Verfahren gefunden werden, das eine hinreichend hohe Reaktionsgeschwindigkeit auch bei tieferen Temperaturen aufweist. Die erhaltenen Polymeren sollten frei von Schwermetallen sein, um sie in der Medizin verwenden zu können. Weiterhin sollte ein Verfahren gefunden werden, das die Herstellung von Blockcopolymeren aus Blöcken aus N-Vinylverbindungen und Blöcken aus anderen Monomeren auf einfache Weise ohne Wechsel des Reaktionsmechanismus ermöglicht.

Demgemäß wurde ein Verfahren zur Herstellung von Polymeren aus N-Vinylverbindungen gefunden, bei dem die Polymerisation der N-Vinylverbindungen in Gegenwart von Radikalen der allgemeinen Formel I 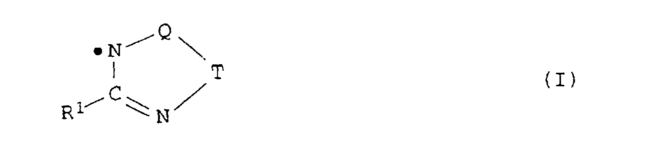 worin Q NR² oder S und T CR³R⁴ oder S bedeutet und R¹, R², R³ und R⁴ gleich oder verschieden voneinander sein können und unabhängig voneinander Wasserstoff, C₁- bis C₂₀-Alkyl oder C₆ - bis C₁₈-Aryl bedeuten, ausgeführt wird. Unter den in der allgemeinen Formel I dargestellten Radikalen sollen erfindungsgemäß auch deren Tautomere und Stellungsisomere verstanden werden. Die Alkylreste können sowohl linear, verzweigt als auch cyclisch sein. Sie konnen sowohl unsubstituiert als auch substituiert sein, beispielsweise mit einem oder mehreren Halogenatomen, wie Chlor, Nitrilgruppen, NO₂, Sulfonsäureresten, Hydroxygruppen, Alkyl- oder Arylesterresten. Darüber hinaus können die Alkylreste Sulfoxid oder Carbonylreste enthalten. Zu den Alkylresten zählen C₁- bis C₁₂-Alkyl, bevorzugt C₁- bis C₁₀-Alkyl, beispielsweise Methyl, Ethyl, n-Propyl, i-Propyl, n-Butyl, i-Butyl, t-Butyl, n-Pentyl, Cyclopentyl, n-Hexyl oder Cyclohexyl. Darunter wird Methyl besonders bevorzugt. Zu den bevorzugten Arylresten zählen Phenyl, Naphtyl und Biphenyl. Die Arylreste können sowohl mit einem oder mehreren Substituenten substituiert als auch unsubstituiert sein. Als Substituenten kommen Alkylreste, beispielsweise C₁- bis C₁₀-Alkyl, bevorzugt C₁- bis C₆-Alkyl wie Methyl, Ethyl, n-Propyl, i-Propyl, n-Butyl, i-Butyl oder t-Butyl oder auch Hydroxygruppen oder Halogenatome, wie Chlor in Betracht. Ferner können die Arylreste auch mit einem oder mehreren Halogenatomen, wie Chlor, Nitrilgruppen, NO₂, Sulfonsäureresten, Alkyl- oder Arylesterresten substituiert sein. Unter den Arylresten ist Phenyl besonders bevorzugt.

Beispiele geeigneter Radikale I sind Thiatianolyle der allgemeinen Formel 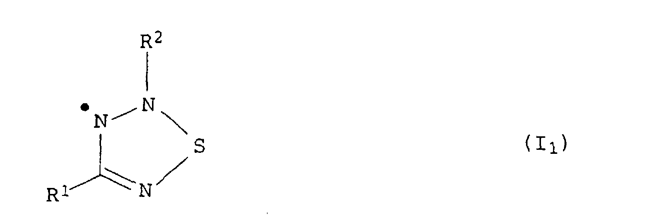 oder Dithiadianolyle der allgemeinen Formel

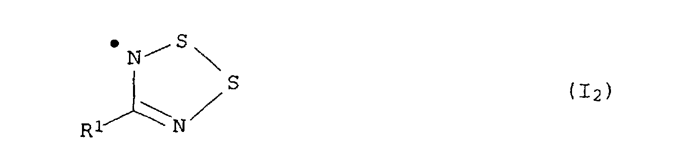

Bevorzugt werden 2,5-Dihydro-1H-1,2,4-triazol-2-ylradikale (Triazoylradikale) der allgemeinen Formel

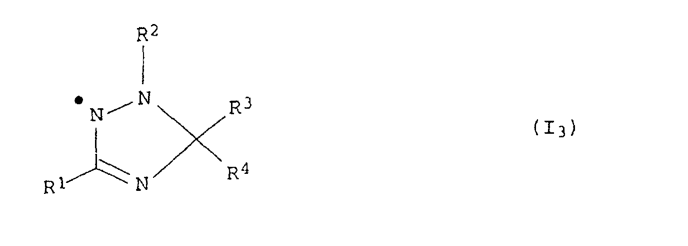

Besonders bevorzugt werden Triazolylradikale, deren Reste R³ und R⁴ gleich sind. Bei den ganz besonders bevorzugten Triazolylradikalen ist R¹ Phenyl, R² Phenyl oder Methyl und R³ und R⁴ jeweils Phenyl, Biphenyl-2,2'-diyl, 6,6'-Dimethylbiphenyl-2,2'- diyl oder 5,5'-Dimethylbiphenyl-2,2'-diyl.

2,5-Dihydro-1H-1,2,4-triazol-2-ylradikale sind an sich bekannt oder nach an sich bekannten Methoden erhältlich. So sind die Triazolylradikale beispielsweise durch Bestrahlen von 1H-1,2,4-Triazolen mit γ-Strahlung zugänglich oder können durch Dehydrogenieren von 4,5-Dihydro-1H-1,2,4-triazolen mit basischer Kaliumhexacyanoferrat-Lösung hergestellt werden. Eine andere Methode um Triazolylradikale zu erhalten, ist die Ringverengung von Tetrazinen in Gegenwart von Säuren (Tetrahedron, 51 (47), 1995, 12883 - 12898).

Thiatrianolyle sind z.B. durch Reduktion der entsprechenden Thiatriazol-1-ium-Salze darstellbar (J. Am. Chem. Soc. Perkin Trans 2 (1990) 1619) erhältlich. Dithiadianolyle sind z.B. durch Reduktion der entsprechenden Dithiadiazadium-Salze erhältlich (Chem. Ber. 118 (1985) 3781.

Die Radikale I können beispielsweise durch eine der vorgenannten Methoden in situ erzeugt werden. Vorzugsweise werden die Radikale I separat hergestellt, isoliert und als solche eingesetzt. Darüberhinaus können die Radikale I in dem erfindungsgemäßen Verfahren auch in Form von Verbindungen II eingesetzt werden, die in Radikalstarter und Radikale I gespalten werden können. Derartige Verbindungen können beispielsweise unter der allgemeinen Formel II zusammengefaßt werden 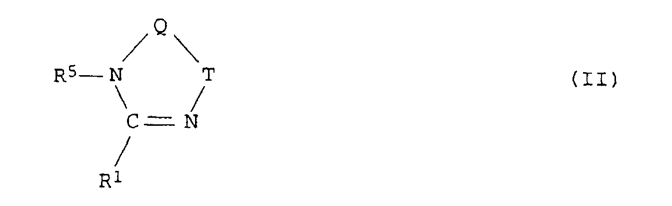 in der R⁵ für einen Rest steht, der, wenn abgespalten, eine radikalische Reaktion initiieren kann. Bevorzugte Verbindungen II enthalten als R⁵ Alkyl, bevorzugt C₁- bis C₁₀-Alkyl, wobei der Alkylrest sowohl linear als auch verzweigt sein kann und durch einen oder mehrere Substituenten, insbesondere Halogenatome, wie Chlor, oder Nitrilgruppen substituiert sein kann. Die Alkylreste können auch durch eine oder mehrere Heteroatome wie Sauerstoff unterbrochen sein. R⁵ kann auch ein Aryl oder substituierter Arylrest, bevorzugt C₆- bis C₁₈-Aryl sein. Bevorzugte Reste R⁵ sind Zerfallsreste handelsüblicher Radikalinitiatoren wie Isobutyronitril oder Benzoyl.

Die Herstellung der Verbindungen II kann z.B. durch Reaktion einer Radikalquelle wie Dibenzoylperoxid oder Azodiisobutyronitril mit einem Radikal I erfolgen. Die Radikalquelle kann dabei nach an sich bekannten Methoden, beispielsweise thermisch, photochemisch oder redorchemisch in die Zerfallsreste gespalten werden.

Die Verbindungen II lassen sich beispielsweise thermisch oder photochemisch spalten. Ferner können die Verbindungen II redoxchemisch gespalten werden. In der Regel werden die Verbindungen II thermisch gespalten. Im allgemeinen spalten die Verbindungen II bei Temperaturen im Bereich von 0 bis 300°C, bevorzugt im Bereich von 50 bis 150°C.

Das erfindungsgemäße Verfahren kann so ausgeführt werden, daß man ein Radikal I bzw eine Verbindung II einsetzt. Ebenso ist es möglich, unterschiedliche Radikale I bzw Verbindungen II einzusetzen. Ferner ist es auch möglich, Mischungen aus Radikalen I und Verbindungen II zu verwenden.

Mit dem erfindungsgemäßen Verfahren lassen sich N-Vinylverbindungen zu Polymeren umsetzen. Bevorzugte N-Vinylverbindungen sind 1-Vinyl-2-pyrrolidon (= N-Vinylpyrrolidon, NVP) und N-Vinylformamid (NVF). N-Vinylpyrrolidon ist ganz besonders bevorzugt.

Sofern sie nicht in Form einer Verbindung II eingesetzt werden, sind die Radikale I im allgemeinen nicht dazu befähigt eine Polymerisationsreaktion zu initiieren. Daher können nach einer der bevorzugten Ausführungsformen Radikalstarter mitverwendet werden. Die Radikalstarter sind an sich bekannt und beispielsweise in Ullmanns Encyclopädie der technischen Chemie, 4. Auflage, Band 15, Seite 187, beschrieben. Besonders geeignet sind Peroxide wie Dibenzoylperoxid und Cumolhydroperoxid und insbesondere Diazoverbindungen wie Azodiisobutyronitril (AIBN). Es können auch Mischungen verschiedener Radikalstarter eingesetzt werden.

Die molare Menge an Radikalstarter kann 10⁻⁶ bis 1 mol/l, vorzugsweise 10⁻⁴ bis 10⁻¹ mol/l, bezogen auf das Volumen der eingesetzten Monomeren, betragen. Das molare Verhältnis von Radikalstarter zu Radikal I beträgt in der Regel von 1 : 0,5 bis 1 : 10, bevorzugt von 1 : 0,5 bis 1 : 5, insbesondere von 1 : 0,5 bis 1 : 2,5.

Nach einer weiteren bevorzugten Ausführungsform können Elektronendonoren, wie sie beispielsweise in der DE-OS 195 16 967 beschrieben werden, mitverwendet werden. Als bevorzugte Elektronendonoren werden Phenothiazin-Derivate oder Phenoselenazine der allgemeinen Formeln 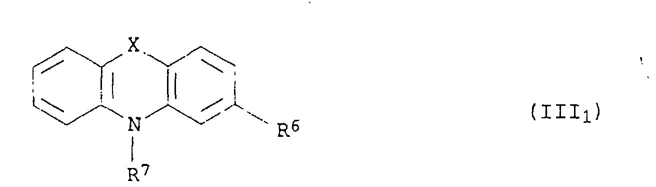 oder 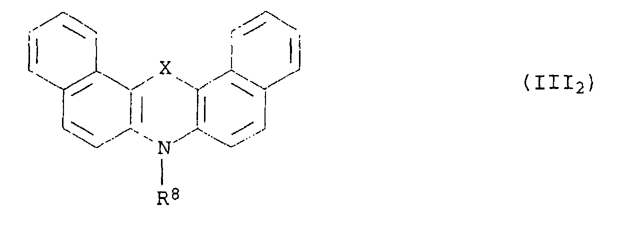 in denen
- X: für Sauerstoff, Schwefel oder Selen, vorzugsweise Schwefel steht
und
- R⁶: ein Wasserstoffatom, -C₁- bis C₅-Alkyl, bevorzugt Methyl oder Ethyl, -CF₃, Halogen, bevorzugt -Cl, -CN, Alkylsulfid, bevorzugt C₁- bis C₁₀-Alkylsulfid, Arylsulfid, bevorzugt Phenylsulfid, Alkoxy, bevorzugt C₁- bis C₁₀-Alkoxy, Aryloxy, bevorzugt Phenoxy, Alkylamin, bevorzugt C₁- bis C₁₀-Alkylamin, Dialkylamin, bevorzugt Di-C₁- bis C₁₀-alkylamin, Arylamin, bevorzugt Phenylamin, Diarylamin, bevorzugt Diphenylamin.
- R⁷: ein Wasserstoffatom oder (̵Z)̵Z¹ ist,
- Z: für eine unverzweigte oder verzweigte C₁- bis C₂₅-Alkylengruppe, bevorzugt eine C₁- bis C₂₅-Alkylengruppe, besonders bevorzugt eine C₁- bis C₁₀-Alkylengruppe, beispielsweise Methylen, Ethylen, 2-Methyl-ethylen, n-Propylen oder n-Butylen steht,
- Z¹: -OH, Alkoxy, bevorzugt C₁- bis C₁₂-Alkoxy, Aryloxy, bevorzugt Phenoxy, Alkylsulfid, bevorzugt C₁- bis C₁₀-Alkylsulfid, Arylsulfid, bevorzugt Phenylsulfid, -NH₂, Alkylamin, bevorzugt C₁- bis C₁₀-Alkylamin, Dialkylamin, bevorzugt Di-C₁- bis C₁₀-alkylamin, Arylamin, bevorzugt Phenylamin, Diarylamin, bevorzugt Diphenylamin oder Z² ist, worunter Z², -NH₂, Alkylamin oder Dialkylamin bevorzugt sind,
- Z²: einen C₄- bis C₇-cycloaliphatischen Rest, vorzugsweise einen C₅- oder C₆-cycloaliphatischen Ring, der eine oder mehrere -O-, -S- oder -N(Alkyl)-Gruppen, bevorzugt -N(C₁- bis C₁₀-Alkylamin) enthalten kann, wobei letztere Gruppe bevorzugt ist und Z² jeweils über ein Kohlenstoffatom mit Z verknüpft ist und die Gruppen -O-, -S- und -N(Alkyl)- nicht direkt miteinander verbunden sind.

Zu den bevorzugten Phenothiazinen zählen: 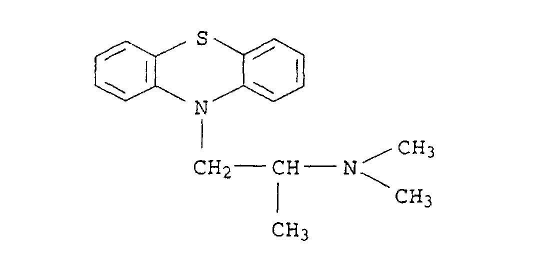 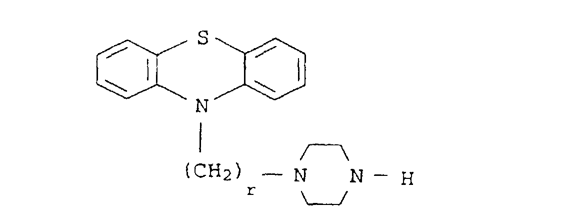 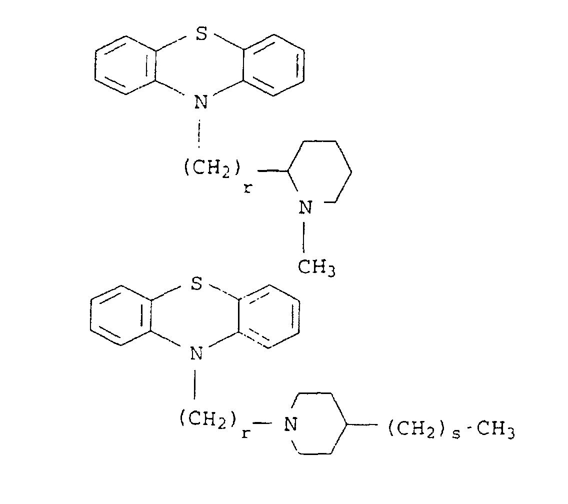 worin r jeweils für eine ganze Zahl von 2 bis 11 steht und s eine ganze Zahl von 1 bis 4 bedeutet.

Es können auch Mischungen verschiedener Elektronendonoren eingesetzt werden.

Die als Elektronendonoren eingesetzten Verbindungen sind an sich bekannt oder nach an sich bekannten Verfahren herstellbar und beispielsweise in J.H. Perlstein, Angew. Chem. Int. Ed. Engl. 16 (1977), Seite 519 bis 534 und M.R. Bryce, Aldrichimica Acta, Vol. 18 (1985), Seite 73 bis 77 beschrieben.

Das molare Verhältnis von Elektronendonoren zu Radikalen I kann im Bereich von 0,1 : 1 bis 10 : 1, vorzugsweise von 0,5 : 1 bis 2 : 1 liegen.

Das molare Verhältnis von Elektronendonoren zu Radikalstartern kann im Bereich von 1:1 bis 3:1, vorzugsweise 1,6:1 bis 2,4:1 liegen.

Das erfindungsgemäße Verfahren kann auch in Gegenwart von Mischungen aus den Radikalen I und N-Oxylradikalen durchgeführt werden. Ferner kommt es in Betracht das erfindungsgemäße Verfahren in Gegenwart von Mischungen aus Radikalen I, Elektronendonoren und N-Oxylradikalen durchzuführen. Dabei dienen die N-Oxylradikale als Moderatoren, d.h. sie verlangsamen die Reaktionsgeschwindigkeit.

N-Oxylradikale sind, wie bereits eingangs erwähnt, an sich bekannt oder sie lassen sich nach an sich bekannten Methoden herstellen. Erfindungsgemäß können N-Oxylradikale unterschiedlichster Struktur eingesetzt werden. Darunter fallen sowohl N-Oxylradikale acyclischer als auch cyclischer Struktur. In der Regel werden cyclische N-Oxylradikale der allgemeinen Formel IV bevorzugt:

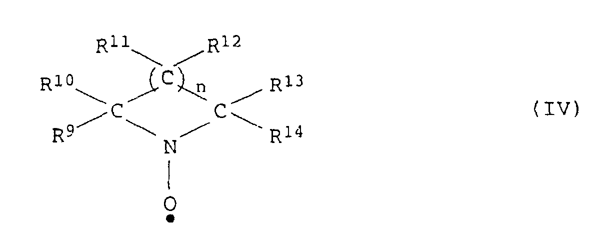

Darin können die Reste R⁹ bis R¹⁴ gleich oder verschieden voneinander sein und bedeuten unabhängig voneinander Wasserstoff, C₁bis C₂₀-Alkyl, C₆- bis C₁₈-Aryl, -OH, -SH, -NH₂, Alkylamin oder Dialkylamin. Die Variable n stellt eine ganze Zahl von 1 bis 5, bevorzugt 2 oder 3 dar. Unter den Alkylresten werden C₁- bis C₁₀-Alkyl, insbesondere C₁- bis C₅-Alkyl und unter den Arylresten Phenyl bevorzugt. Bevorzugt sind die Reste R⁹ und R¹⁰ sowie R¹³ und R¹⁴ jeweils Phenyl oder Alkyl oder ein Phenylrest und eine Alkylgruppe wie Methyl oder Ethyl. R¹¹ und R¹² sind bevorzugt Wasserstoff. Ist n größer als 1 können die CR¹¹R¹²-Gruppen jeweils gleich sein. Es können aber auch unterschiedliche CR¹¹R¹²-Gruppen enthalten sein. Bei mehr als einer CR¹¹R¹²-Gruppe bedeuten die Reste einer dieser Gruppen bevorzugt OH und Wasserstoff.

Bevorzugt sind 2,2,6,6-Tetramethyl-1-piperidinyloxy (TEMPO), 4-Oxo-2,2,6,6-tetramethyl-1-piperidinyloxy (4-Oxo-TEMPO), 4-Hydroxy-2,2,6,6-tetramethyl-1-piperidinyloxy, 2,2,5,5-tetramethyl-1-pyrrolidinyloxy, 3-Carboxy-2,2,5,5-tetramethyl-pyrrolidinyloxy und Di-tert.-butylnitroxid. 2,6-Diphenyl-2,6-dimethyl-1-piperidinyloxy sowie 2,5-Diphenyl-2,5-dimethyl-1-pyrrolidinyloxy können ebenfalls eingesetzt werden. Mischungen verschiedener N-Oxyl-Radikale können auch eingesetzt werden.

Das molare Verhältnis von N-Oxylradikal und Radikal I liegt im allgemeinen im Bereich von 0,1 : 1 bis 20 : 1, bevorzugt im Bereich von 0,1 : 1 bis 10 : 1, besonders bevorzugt im Bereich von 0,1 : 1 bis 2 : 1.

Das molare Verhältnis zwischen dem N-Oxylradikal und dem Radikalstarter liegt vorzugsweise im Bereich von 0,5:1 bis 5:1, insbesondere von 0,8:1 bis 4:1, besonders bevorzugt im Bereich von 1 : 1 bis 1,5 : 1.

Die erfindungsgemäße Polymerisation kann nach unterschiedlichsten Methoden wie Masse-, Lösungs-, Emulsions- oder Suspensionspolymerisation vorgenommen werden. Beispielsweise kann die Polymerisation in der Schmelze, z.B. in einem Extruder oder einem Kneter durchgeführt werden. Als Lösungsmittel für die Polymerisation in Lösung eignen sich beispielsweise Tetrahydrofuran, Toluol, Ethylbenzol oder deren Mischungen.

Die Reaktionsbedingungen sind in der Regel unkritisch, die Temperaturen können im Bereich von 0 bis 220°C, vorzugsweise im Bereich von 20 bis 180°C liegen, üblicherweise arbeitet man bei Normaldruck, man kann aber auch bei Drücken bis zu 30 bar arbeiten. Die Reaktionszeiten wählt man vorzugsweise so, daß man polymerisiert, bis das gewünschte Molekulargewicht erreicht wird, beispielsweise 1 Stunde bis 6 Tage.

Es kann vorteilhaft sein, die Reaktion unter Inertgas, beispielsweise Stickstoff oder einem Edelgas wie Argon, durchzuführen.

Bei dem erfindungsgemäßen Verfahren geht man vorzugsweise so vor, daß man den Radikalstarter und das Radikal I oder eine Verbindung II vorlegt und das Monomer oder die Monomere und gegebenenfalls das Lösungsmittel hinzugibt. Möglich ist jedoch auch eine umgekehrte Reihenfolge der Zugabe. Werden Elektronendonoren oder N-Oxylradikale oder deren Mischungen mitverwendet, können diese zusammen mit den Radikalstartern und dem Radikal I vorgelegt werden. Sie können aber auch getrennt oder einzeln im Verlauf der Polymerisationsreaktion zugegeben werden. Die Aufarbeitung der Polymerisate kann durch Ausfällen, beispielsweise in Methanol oder Hexan, erfolgen.

Die Molekulargewichte Mₙ (Zahlenmittelwert) der entstehenden Polymeren können in weiten Bereichen schwanken, beispielsweise von 5000 bis 500.000 g/mol.

Mittels des erfindungsgemäßen Verfahrens lassen sich neben Homopolymeren auch statistische Copolymere herstellen. Statistische Copolymere werden zweckmäßigerweise hergestellt, indem man die N-Vinylverbindungen zusammen mit geeigneten ungesättigten Monomeren, insbesondere vinylischen Monomeren, polymerisiert.

Als vinylische Comonomere eignen sich besonders Olefine, Vinylchlorid, Vinylidenchlorid, Ester aus Vinylalkohol und Monocarbonsäuren mit 1 bis 8 C-Atomen, wie z.B. Vinylacetat, Vinylaromaten wie Styrol, 2-Vinylnaphthalin und 9-Vinylanthracen, substituierte Vinylaromaten wie p-Methylstyrol, α-Methylstyrol, p-Chlorstyrol, 2,4-Dimethylstyrol und 4-Vinylbiphenyl, C₁- bis C₈-Alkylester der Acrylsäure oder der Methacrylsäure, besonders C₁- bis C₄-Acrylate und C₁- bis C₄-Methacrylate, ungesättigte Dicarbonsäuren, beispielsweise aliphatische ungesättigte Dicarbonsäuren wie Maleinsäure, Fumarsäure und Itaconsäure, oder deren Derivate wie Anhydride, Ester und Amide, insbesondere Anhydride, wie Maleinsäureanhydrid, oder Vinylcyanide, insbesondere Acrylnitril. Mischungen verschiedener Comonomere können ebenfalls eingesetzt werden.

Bevorzugte Comonomere sind Styrol, substituierte Styrole, C₁- bis C₄-Acrylate und C₁- bis C₄-Methacrylate, insbesondere Methylmethacrylat, sowie Acrylnitril.

Der Anteil der Comonomeren beträgt üblicherweise bis zu 80, bevorzugt bis zu 50, besonders bevorzugt bis zu 30 Gew.-%, bezogen auf das erhaltene Copolymere aus N-Vinylverbindungen und Comonomer.

Neben den beschriebenen Homo- und statistischen Copolymeren lassen sich mit dem erfindungsgemäßen Verfahren auch segmentierte Copolymere wie Blockcopolymere, Sternblockcopolymere, Pfropfcopolymere oder Pfropfblockcopolymere herstellen, indem die Polymeren vorzugsweise ohne Aufarbeitung mit anderen Monomeren oder Monomermischungen anderer Zusammensetzung weiter umgesetzt werden. Hierbei kann die Zugabe weiterer Mengen an Radikalen I oder Verbindungen II, Radikalstartern, Elektronendonoren oder N-Oxylradikalen oder deren Mischungen erforderlich sein.

Nach dem erfindungsgemäßen Verfahren können auch Blockcopolymere hergestellt werden aus
- wenigstens einem Polymerblock A erhältlich durch Polymerisation in Gegenwart von Radikalen I aus NVP-Homopolymer oder NVP-Copolymer mit bis zu 80, bevorzugt bis zu 50 Gew.-%, bezogen auf den Block A, Comonomeren
   und
- wenigstens einem Polymerblock B erhältlich durch Polymerisation in Gegenwart von Radikalen I aus NVF-Homopolymer oder NVF-Copolymer mit bis zu 80, bevorzugt bis zu 50 Gew.-%, bezogen auf den Block B, Comonomeren,
wobei die Polymerblöcke A, B direkt und nicht durch konstitutionelle Einheiten, die nicht Teil der Blöcke sind, miteinander verbunden sind.

Als bevorzugte Comonomere für Block A und B seien die zuvor bereits erwähnten vinylischen Comonomere, sowie die weiter unten für den Block C genannten Monomere, genannt.

Symbolisiert man einen Polymerblock der Sorte A mit A und einen Polymerblock der Sorte B mit B und läßt man Initiator-, gegebenenfalls Moderator- und Abbruchreste unberücksichtigt, so kommen als erfindungsgemäße amphiphile Blockcopolymere beispielsweise in Betracht: lineare Systeme wie A-B, A-B-A, B-A-A, B-A-E, A-B-B oder (A-B)ₙ, sternförmige Systeme wie A(B)ₙ, B(A)ₙ oder (A)ₙ-B-A- (B)ₘ, dendrimere Systeme wie ((A)ₙ-B)ₘA, ((B)ₙ-A)ₘB, ((A)ₘ-B)ₙA)ₚB oder ((B)ₘ-A)ₙB)ₚA oder kammförmige Systeme wie ((A)ₙ-A(B))_{q} oder ((B)ₙ-B(A))_{q}, wobei m, n und p ganze Zahlen von 1 bis 5 bedeuten und q eine ganze Zahl von 0 bis 1000.

Ferner sind erfindungsgemäß lineare Di- und Triblockcopolymere bevorzugt. Gibt die Abfolge der Buchstaben A,B die zeitliche Abfolge der Blockherstellung wieder, lassen sich erfindungsgemäß günstige Blockcopolymere schematisch darstellen als A-B, B-A, B-A-B und A-B-A.

Nach dem erfindungsgemäßen Verfahren können auch sog. isophile Blockcopolymere hergestellt werden, also Blockcopolymere, deren Blöcke zwar aus verschiedenen Monomeren bestehen, jedoch im gleichen Lösungsmittel eine vergleichbare oder zumindest ähnliche Löslichkeit aufweisen. Gemäß der Erfindung bestehen diese isophilen Blockcopolymeren aus
- wenigstens einem Polymerblock A aus NVP-Homo- oder Copolymer, wie er oben bereits definiert wurde
   und/oder
- wenigstens einem Polymerblock B aus NVF-Homo- oder Copolymer, wie er oben bereits definiert wurde,
   und
- wenigstens einem Polymerblock C, erhältlich durch Polymerisation in Gegenwart von Radikalen I von einem oder mehreren hydrophilen Monomeren aus der Gruppe bestehend aus Acrylsäure, Methacrylsäure, Maleinsäure, Fumarsäure, Itaconsäure, 2-Acrylamido-2-methylpropansulfonsäure, Styrolsulfonsäure, die Kalium-, Natrium- und Ammoniumsalze sowie die Amide der vorgenannten Säuren, ω-Hydroxy-C₂- bis C₄-alkylacrylat, ω-Hydroxy-C₂- bis C₄-alkylmethacrylat, Vinylimidazol, Vinylcaprolactam, N-Methylvinylimidazol, Vinylmethylether und Dimethylaminoethylacrylat,
wobei die Polymerblöcke der Sorten A,B,C direkt und nicht durch konstitutionelle Eiheiten, die nicht Teil der Blöcke sind, miteinander verbunden sind.

Die isophilen Blockcopolymere enthalten demnach immer den Block C und entweder den Block A oder den Block B oder die Blöcke A, B und C.

Symbolisiert man einen Polymerblock der Sorte A mit A und einen polymerblock der Sorte C mit C und läßt man Initiator-, gegebenenfalls Moderator- und Abbruchreste unberücksichtigt, so komm als erfindungsgemäße amphiphile Blockcopolymere beispielsweise Betracht: lineare Systeme wie A-C, A-C-A, C-A-A, C-A-C, A-C-C oder (A-C)ₙ, sternförmige Systeme wie A(C)ₙ, C(A)ₙ oder (A)ₙ-C-A-(C)ₘ, dendrimere Systeme wie ((A)ₙ-C)ₘA, ((C)ₙ-A)ₘC, ((A)ₘ-C)ₙA)ₚC oder ((C)ₘ-A)ₙC)ₚA oder kammförmige Systeme wie ((A)ₙ-A(C))_{q} oder ((C)ₙ-C(A))_{q}, wobei m, n und p ganze Zahlen von 1 bis 5 bedeuten und q eine ganze Zahl von 0 bis 1000.

Ferner sind erfindungsgemäß lineare Di- und Triblockcopolymere bevorzugt. Gibt die Abfolge der Buchstaben A, C die zeitliche Abfolge der Blockherstellung wieder, lassen sich erfindungsgemäß günstige amphiphile Blockcopolymere schematisch darstellen als A-C, C-A, C-A-C und A-C-A.

Die gleichen Blockabfolgen gelten sinngemäß auch für die Blöcke B und C: in den vorstehenden schematischen Blockfolgen ist jeweils A durch B zu ersetzen.

Der Block C kann mit den Blöcken A und B in jeder beliebigen Reihenfolge verknüpft sein, z.B. A-B-C, A-C-B, C-A-B, etc.

Für alle vorgenannten Blockcopolymeren aus den Blöcken A und B, oder A und C, oder B und C, oder A, B und C gilt: Die erfindungsgemäßen Blockcopolymere (der Begriff Blockcopolymer steht hier für Polymere, deren Moleküle aus vorzugsweise linear verknüpften Blöcken bestehen, wobei die Blöcke direkt miteinander verbunden sind und wobei der Begriff Block einen Abschnitt eines Polymermoleküls meint, der mehrere monomere Einheiten umfaßt, die wenigstens ein gemeinsames Merkmal besitzen, das in den unmittelbar angrenzenden Abschnitten nicht auftritt) können Zweiblockcopolymere, Dreiblockcopolymere oder auch mehr als drei Blöcke umfassende Multiblockcopolymere sein. Vorzugsweise sind sie unvernetzt.

Zu im wäßrigen Medium löslichen erfindungsgemäßen Blockcopolymeren sollen auch diejenigen gerechnet werden, die zwar nicht auf direktem Weg im wäßrigen Polymerisationsmedium löslich sind, deren Lösung jedoch indirekt z.B. dadurch bewirkt werden kann, daß man sie zunächst in einem mit Wasser mischbaren organischen Lösungsmittel bzw. in einem Gemisch aus Wasser und einem solchen organischen Lösungsmittel löst (z.B. in Dioxan, Tetrahydrofuran oder deren Gemischen mit Wasser) und diese Lösung (die erfindungsgemäß teilweise auch unmittelbar ins wäßrige Polymerisationsmedium zugesetzt werden kann) anschließend z.B. via Dialyse oder mehrfachen Zusatz kleiner Wassermengen und nachfolgende destillative Abtrennung des verwendeten organischen Lösungsmittels in eine wäßrige Lösung wandelt (anstelle von Wasser wird häufig auch eine wäßrige Lösung einer Säure und/oder Base angewendet). Der Begriff Lösung impliziert hier nicht notwendigerweise eine molekulare Lösung, sondern bringt lediglich zum Ausdruck, daß es sich um eine klare Flüssigkeit handelt und umfaßt insbesondere auch micellare Lösungen, insbesondere auch solche, die sich nicht im thermodynamischen Gleichgewicht befinden.

Die erfindungsgemäßen Blockcopolymere weisen in der Regel am Kettenende einen Rest auf, der sich von den Radikalen I bzw. von R⁵ ableitet. Teilweise können diese Reste durch eine endständige Oxyamingruppe ausgetauscht sein. Aus verschiedenen Gründen kann eine Beseitigung der Reste, die sich von den Radikalen I ableiten, erwünscht sein. In Spalte 6, Zeilen 54ff, bietet die US-A 4,581,429 verschiedene solcher Beseitigungsmöglichkeiten an. Erfindungsgemäß von besonderem Interesse sind diejenigen, die zu einem -H, einer Hydroxylgruppe oder zu einer ethylenisch ungesättigten endständigen Gruppe führen.

Das erfindungsgemäße Verfahren zeichnet sich insbesondere durch Wirtschaftlichkeit aus, da die Reaktion bei dem technisch interessanten Temperaturbereich hinreichend schnell abläuft und sich die Reaktionsgeschwindigkeit außerdem gut steuern läßt. Das erfindungsgemäße Verfahren ist weitgehend unempfindlich gegen geringe Mengen an Feuchtigkeit und es können auch Gemische von Monomeren zu statistischen Copolymerisaten umgesetzt werden.

Die erfindungsgemäße Verwendung der Radikale I bzw.
Verbindungen II führt zu Polymeren mit reaktiven "lebenden" radikalischen Kettenenden, so daß durch einfache Zugabe eines anderen radikalisch polymerisierbaren Monomeren(gemisches) zum Reaktor ein Blockcopolymer oder ein anderes Copolymer wie z.B. Sternblockcopolymer, Pfropfcopolymer oder Pfropfblockcopolymer erhalten werden kann. Eine Isolation des anfangs hergestellten Polymeren mit lebendem Kettenende ist ebensowenig erforderlich wie die schwierige und zeitaufwendige "Umpolung" des Reaktivitätszentrums, wie sie aus der klassischen Blockcopolymerisation bekannt ist. Blockcopolymere oder andere Copolymere, enthaltend N-Vinylverbindungen, können nach dem erfindungsgemäßen Verfahren demnach bequem in einer "Ein-Topf-Reaktion" hergestellt werden. Das erfindungsgemäße Verfahren erlaubt auch die einfache Herstellung von Blockcopolymeren aus Monomeren, die anionisch und/oder kationisch nicht polymerisiert werden können.

Die erhaltenen Polymere der N-Vinylverbindungen sind frei von Schwermetallen. Die polymerisierten N-Vinylverbindungen und insbesondere PolyNVP-Homopolymer, das nach dem erfindungsgemäßen Verfahren hergestellt wird, eignen sich daher besonders für die Verwendung in der Medizin. Durch Einstellung der Polymerisationsbedingungen kann insbesondere PolyNVP mit einem Molekulargewicht hergestellt werden, welches die menschliche Niere passieren kann. Ein solches PolyNVP ist daher als Blutplasmaersatz geeignet.

### Beispiele

Kommerziell erhältliches Benzoylperoxid wurde ohne weitere Reinigung eingesetzt. 2,5-Dihydro- 1,3,5,5-tetraphenyl-1H-1,2,4,-triazol-2-yl wurde gemäß Tetrahedron 51(47), 12883-12898, 1995 durch Dehydrogenierung des entsprechenden 4,5-Dihydro-1H-1,2,4-triazols hergestellt.

### Vergleichsversuch: Polymerisation von NVP ohne Radikal I

In einem Rührkessel-Reaktor wurden 500 g N-Vinylpyrrolidon und 1 g Benzoylperoxid als radikalischer Initiator vorgelegt. Nach Spülen mit Stickstoffgas wurde unter Rühren auf 130°C aufgeheizt und 5 Stunden bei dieser Temperatur gehalten. Nach dem Abkühlen wurde das erhaltene Polymere durch Eingießen der Reaktionsmischung in Cyclohexan ausgefällt und getrocknet.

Das erhaltene PolyNVP hatte ein Molekulargewicht (Gewichtsmittel) von 120 000 g/mol; der Polydispersitätsindex PDI = Gewichtsmittel/Zahlenmittel betrug 8,4.

### Versuch:

Es wurde verfahren wie im Vergleichsversuch, jedoch wurde zusammen mit NVP und Benzoylperoxid 1,2 g 1,3,5,5-Tetraphenyl-2,5-dihydro-1H-1,2,4-triazol-2-yl (Formel I₃ mit R¹ bis R⁴ = Phenyl) vorgelegt.

Das erhaltene PolyNVP hatte ein Molekulargewicht (Gewichtsmittel) von 90 000, der Polydispersitätsindex PD) betrug 5,8.

Die Versuche zeigen, daß die erfindungsgemäße Mitverwendung der Radikale I zu Poly-N-Vinylverbindungen mit geringerer Polydispersität und geringerem Molekulargewicht führt.

## Patentansprüche

1. Verfahren zur Herstellung von Polymeren aus N-Vinylverbindungen, **dadurch gekennzeichnet, daß** man die Polymerisation der N-Vinylverbindungen in Gegenwart von Radikalen der allgemeinen Formel I 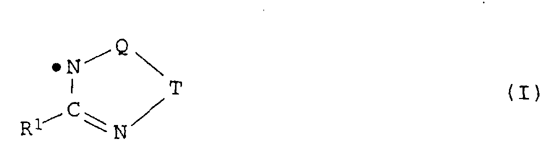 worin Q NR² oder S und T CR³R⁴ oder S bedeutet und R¹, R², R³ und R⁴ gleich oder verschieden voneinander sein können und unabhängig voneinander Wasserstoff, C₁- bis C₂₀-Alkyl oder C₆bis C₁₈-Aryl bedeuten, durchführt.

2. Verfahren nach Anspruch 1, **dadurch gekennzeichnet, daß** man einen Radikalstarter oder einen Elektronendonor oder deren Mischungen mitverwendet.

3. Verfahren nach den Ansprüchen 1 bis 2, **dadurch gekennzeichnet, daß** man als Radikale I 2,5-Dihydro-1H-1,2,4- triazolylradikale einsetzt.

4. Verfahren nach den Ansprüche 1 bis 3, **dadurch gekennzeichnet, daß** man die Radikale I aus Verbindungen II, die in Radikalstarter und Radikale I gespalten werden können, erzeugt.

5. Verfahren nach den Ansprüchen 1 bis 4, **dadurch gekennzeichnet, daß** man als N-Vinylverbindung 1-Vinyl-2-pyrrolidon (N-Vinylpyrrolidon) oder N-Vinylformamid oder deren Mischung einsetzt.

6. Verwendung von Radikalen I zur Herstellung von Polymeren aus N-Vinylverbindungen.

7. Verwendung der gemäß einem der Ansprüche 1 bis 5 hergestellten Polymeren aus N-Vinylverbindungen zur Herstellung von Blutplasmaersatz.

8. Polymere aus N-Vinylverbindungen, erhältlich nach dem Verfahren gemäß einem der Ansprüche 1 bis 5.

9. Blockcopolymere, erhaltlich durch Umsetzen von
wenigstens einem Polymerblock A erhältlich durch Polymerisation in Gegenwart von Radikalen I aus N-Vinylpyrrolidon-Homopolymer oder N-Vinylpyrrolidon-Copolymer mit bis zu 80 Gew.-%, bezogen auf den Block A, Comonomeren
und
- wenigstens einem Polymerblock B erhältlich durch Polymerisation in Gegenwart von Radikalen I aus N-Vinylformamid-Homopolymer oder N-Vinylformamid-Copolymer mit bis zu 80 Gew.-%, bezogen auf den Block B, Comonomeren,
wobei die Polymerblöcke A, B direkt und nicht durch konstitutionelle Einheiten, die nicht Teil der Blöcke sind, miteinander verbunden sind, nach dem Verfahren gemäß den Ansprüchen 1 bis 5.

10. Blockcopolymere, erhältlich durch Umsetzen von
- wenigstens einem Polymerblock A aus N-Vinylpyrrolidon-Homo- oder Copolymer wie in Anspruch 9 definiert
und/oder
- wenigstens einem Polymerblock B aus N-Vinylformamid-Homooder Copolymer wie in Anspruch 9 definiert
und
- wenigstens einem Polymerblock C, erhältlich durch Polymerisation in Gegenwart von Radikalen I von einem oder mehreren hydrophilen Monomeren aus der Gruppe bestehend aus Acrylsäure, Methacrylsäure, Maleinsäure, Fumarsäure, Itaconsäure, 2-Acrylamido-2-methylpropansulfonsäure, Styrolsulfonsäure, die Kalium-, Natrium- und Ammoniumsalze sowie die Amide der vorgenannten Säuren, ω-Hydroxy-C₂- bis C₄ - alkylacrylat, ω-Hydroxy-C₂- bis C₄-alkylmethacrylat, Vinylimidazol, Vinylcaprolactam, N-Methylvinylimidazol, Vinylmethylether und Dimethylaminoethylacrylat,
wobei die Polymerblöcke der Sorten A,B,C direkt und nicht durch konstitutionelle Einheiten, die nicht Teil der Blöcke sind, miteinander verbunden sind, nach dem Verfahren gemäß den Ansprüchen 1 bis 5.

## Claims

1. A process for preparing polymers of N-vinyl compounds, which comprises polymerizing the N-vinyl compounds in the presence of free radicals of the formula I 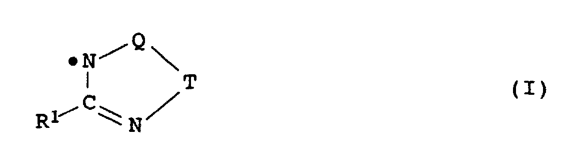 where Q is NR² or S and T is CR³R⁴ or S and R¹, R², R³ and R⁴ can be identical or different and are, independently of one another, hydrogen, C₁-C₂₀-alkyl or C₆-C₁₈-aryl.

2. A process as claimed in claim 1, wherein a free-radical initiator or an electron donor or a mixture thereof is also used.

3. A process as claimed in claim 1 or 2, wherein the free radicals I used are 2,5-dihydro-1H-1,2,4-triazolyl radicals.

4. A process as claimed in any of claims 1 to 3, wherein the free radicals I are generated from compounds II which can be dissociated into free-radical initiators and free radicals I.

5. A process as claimed in any of claims 1 to 4, wherein the N-vinyl compound used is 1-vinyl-2-pyrrolidone (N-vinylpyrrolidone) or N-vinylformamide or a mixture thereof.

6. The use of free radicals I for preparing polymers of N-vinyl compounds.

7. The use of a polymer of N-vinyl compounds prepared as claimed in any of claims 1 to 5 for producing a blood plasma substitute.

8. A polymer of N-vinyl compounds obtainable by a process as claimed in any of claims 1 to 5.

9. A block copolymer obtainable by reacting
- at least one polymer block A obtainable by polymerization in the presence of free radicals I and consisting of N-vinylpyrrolidone homopolymer or N-vinylpyrrolidone copolymer comprising up to 80% by weight, based on the block A, of comonomers
and
- at least one polymer block B obtainable by polymerization in the presence of free radicals I and consisting of N-vinylformamide homopolymer or N-vinylformamide copolymer comprising up to 80% by weight, based on the block B, of comonomers,
where the polymer blocks A, B are joined to one another directly and not via structural units which are not part of the blocks, according to a process as claimed in any of claims 1 to 5.

10. A block copolymer obtainable by reacting
- at least one polymer block A consisting of N-vinylpyrrolidone homopolymer or copolymer as defined in claim 9
and/or
- at least one polymer block B consisting of N-vinylformamide homopolymer or copolymer as defined in claim 9
and
- at least one polymer block C obtainable by polymerization in the presence of free radicals I of one or more hydrophilic monomers selected from the group consisting of acrylic acid, methacrylic acid, maleic acid, fumaric acid, itaconic acid, 2-acrylamido-2-methylpropanesulfonic acid, styrenesulfonic acid, the potassium, sodium and ammonium salts and also the amides of the abovementioned acids, ω-hydroxy-C₂-C₄-alkyl acrylate, ω-hydroxy-C₂-C₄-alkyl methacrylate, vinylimidazole, vinylcaprolactam, N-methylvinylimidazole, vinyl methyl ether and dimethylaminoethyl acrylate,
where the polymer blocks of the types A, B, C are joined to one another directly and not via structural units which are not part of the blocks, according to a process as claimed in any of claims 1 to 5.

## Revendications

1. Procédé de préparation de polymères à partir de composés N-vinyliques, **caractérisé en ce que** l'on procède à la polymérisation des composés N-vinyliques en présence de radicaux libres de formule générale I 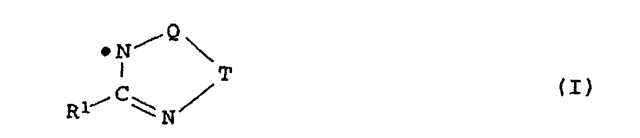 dans laquelle Q représente un groupe NR² ou un atome de S et T représente un groupe CR³R⁴ ou un atome de S, et R¹, R², R³ et R⁴ peuvent être identiques ou différents les uns des autres et représentent, indépendamment les uns des autres, un atome d'hydrogène, un groupe alkyle en C₁ à C₂₀ ou un groupe aryle en C₆ à C₁₈.

2. Procédé selon la revendication 1, **caractérisé en ce que** l'on utilise, conjointement, un amorceur radicalaire ou un donneur d'électron, ou leurs mélanges.

3. Procédé selon les revendications 1 à 2, **caractérisé en ce que** l'on met en oeuvre, en tant que radicaux libres I, des radicaux 2,5-dihydro-1H-1,2,4-triazolyle.

4. Procédé selon les revendications 1 à 3, **caractérisé en ce que** l'on produit les radicaux libres I à partir de composés II, qui peuvent être décomposés en amorceur radicalaire et en radicaux libres I.

5. Procédé selon les revendications 1 à 4, **caractérisé en ce que** l'on met en oeuvre , en tant que composé N-vinylique, la 1-vinyl-2-pyrrolidone (N-vinylpyrrolidone) ou le N-vinylformamide ou leur mélange.

6. Mise en oeuvre de radicaux libres I pour la préparation de polymères à partir de composés N-vinyliques.

7. Mise en oeuvre des polymères préparés selon l'une quelconque des revendications 1 à 5, à partir de composés N-vinyliques, pour la préparation de produits de substitution de plasma sanguin.

8. Polymères à base de composés N-vinyliques, que l'on peut obtenir au moyen du procédé selon l'une quelconque des revendications 1 à 5.

9. Polymères séquencés que l'on peut obtenir en faisant réagir
- au moins une séquence polymère A pouvant être obtenue par polymérisation, en présence de radicaux libres I, d'homopolymère de la N-vinylpyrrolidone ou de copolymère de la N-vinylpyrrolidone avec jusqu'à 80% en poids, par rapport à la séquence A, de comonomères, et
- au moins une séquence polymère B pouvant être obtenue par polymérisation, en présence de radicaux libres I d'homopolymère du N-vinylformamide ou de copolymère du N-vinylformamide, avec jusqu'à 80% en poids, par rapport à la séquence B, de comonomères,
les séquences polymères A, B étant liées entre elles directement, et pas par l'intermédiaire de motifs constitutionnels ne faisant pas partie des séquences, au moyen du procédé selon les revendications 1 à 5.

10. Copolymères séquences, que l'on peut obtenir en faisant réagir :
- au moins une séquence polymère A composée d'homo- ou de copolymère de la N-vinylpyrrolidone, telle que définie dans la revendication 9, et/ou
- au moins une séquence polymère B composée d'homo- ou de copolymère du N-vinylformamide, telle que définie dans la revendication 9, et
- au moins une séquence polymère C, pouvant être obtenue par polymérisation, en présence de radicaux libres I, d'un ou de plusieurs monomères hydrophiles du groupe formé par l'acide acrylique, l'acide méthacrylique, l'acide maléique, l'acide fumarique, l'acide itaconique, l'acide 2-acrylamido-2-méthylpropanesulfonique, l'acide styrolsulfonique, les sels de potassium, de sodium et d'ammonium ainsi que les amides des acides susmentionnés, l'acrylate de ω-hydroxy(alkyle en C₂ à C₄), le méthacrylate de ω-hydroxy(alkyle en C₂ à C₄), le vinylimidazole, le vinylcaprolactame, le N-méthylvinylimidazole, l'éther vinylméthylique et l'acrylate de diméthylaminoéthyle,
les séquences polymères de types A, B, C étant liées entre elles directement, et pas par l'intermédiaire de motifs constitutionnels ne faisant pas partie des séquences, au moyen du procédé selon les revendications 1 à 5.
